# EUROPEAN PATENT SPECIFICATION

(11) **EP 0 576 742 B1**
(45) Date of publication and mention of the grant of the patent: **09.09.1998**
(21) Application number: 92305125.4
(22) Date of filing: 04.06.1992
(51) Int. Cl.: C12Q 1/68

(54) **Nucleic acid probes for the detection of genital mycoplasmas**
Nucleinsäureproben zum Nachweis genitaler Mycoplasmen
Sondes d'acides nucléiques pour la détection des mycoplasmes génitaux

(43) Date of publication of application: 05.01.1994
(73) Proprietor: VYSIS, Inc., Downers Grove, Illinois 60515-5424 (US)
(72) Inventor: Weisburg, William Greene, Milford, MA 01757 (US); Pelletier, Dale Adam, Brighton, MA 02135 (US)
(74) Representative: Hall, Marina

(56) References cited:
- EP-A- 0 250 662
- WO-A-88/03957
- WO-A-90/15157
- FEMS MICROBIOLOGY LETTERS vol. 81, no. 1, 1 June 1991, AMSTERDAM NL pages 37 - 42 A.BLANCHARD ET AL. 'Detection and identification of mycoplasmas by amplification of rDNA'
- JOURNAL OF BACTERIOLOGY vol. 171, no. 12, December 1989, BALTIMORE US pages 6455 - 6467 W.G.WEISBURG ET AL. 'A phylogenetic analysis of the mycoplasmas: basis for their classification'
- NUCLEIC ACIDS RESEARCH vol. 19, 1991, LONDON GB pages 6027 - 6031 S.N.PETERSON ET AL. 'A random sequencing approach for placing markers on the physical map of Mycoplasma genitalium'
- MICROBIOLOGY AND IMMUNOLOGY vol. 36, no. 1, January 1992, TOKYO JP pages 21 - 27 Y.SASAKI ET AL 'Detection and discrimination of Mycoplasma pneumoniae and Mycoplasma genitalium by the in vitro DNA amplification'
- APPLIED AND ENVIRONMENTAL MICROBIOLOGY vol. 58, no. 8, August 1992, WASHINGTON US pages 2606 - 2615 F.J.M.VAN KUPPEVELD ET AL. 'Genus- and species-specific identification of mycoplasmas by 16s rRNA amplification'

## Description

### Field of the Invention

This invention relates to nucleic acid probes for the detection of mycoplasmas associated with the genito-urinary tract, believed to be the cause of nongonococcal urethritis, pelvic inflammatory diseases, septic abortion, and a wide array of diseases of other tissues. More specifically, embodiments of the present invention provide nucleic acid probes and compositions, methods for their use for the specific detection or identification of Mycoplasma hominis, Ureaplasma urealyticum, and Mycoplasma genitalium in clinical and other samples, and packaged components suitable for use as kits.

### Background of the Invention

Mycoplasmas are small wall-less bacteria, primarily isolated from animal sources including humans. There are over 70 members of the genus Mycoplasma, and several related genera which are also characterized by small wall-less bacteria; these are Spiroplasma, Acholeplasma, Ureaplasma, Anaeroplasma, and Asteroleplasma. Only a handful of the species within these genera have been found associated with humans--some presumed to be "normal flora", others occasionally pathogenic, and still others always believed to be clinically significant. Among the mycoplasmas known to be pathogenic, Mycoplasma pneumoniae is historically the most well studied, it is the major infectious agent of primary atypical pneumonia.

Three other mycoplasma species, which can be isolated from the human genito-urinary tract, Mycoplasma hominis, Mycoplasma genitalium, and Ureaplasma urealyticum, are somewhat more enigmatic in the clinical implications of the detection of these organisms in the human body. Ureaplasma urealyticum, for example. although it is implicated in significant and serious human morbidity and mortality, may be found in asymptomatic "normal" individuals as well. The three species, Mycoplasma hominis, Mycoplasma genitalium, and Ureaplasma urealyticum will be referred to herein as the genital mycoplasmas.

EP-A-0 250 662 describes a method for detecting mycoplasmas or prokaryotes using nucleic acid probes.

Mycoplasma genitalium was initially isolated from the urethras of two males with nongonococcal urethritis (Tully, et.al., Int. J. Syst. Bacteriol. vol. 33, 1983). Subsequently, M. genitalium was isolated from the human respiratory tract, in co-culture with Mycoplasma pneumoniae (Baseman, et.al., J. Clinical Micro. vol.26, 1988). However, studies have shown (for example Hooton, et.al., Lancet, 1988) that M. genitalium plays a role in at least some of the cases of acute urethritis, particularly in homosexual males. It is an aspect of the present invention to describe methods for the design and manufacture of probes specific for the detection of Mycoplasma genitalium. Nucleic acid probes which recognize both M. genitalium and Mycoplasma pneumoniae, evolutionary close relatives, are described in an application filed concurrently.

The article by Y. Sasaki et al. in Microbiol. Immunol., Vol. 36 (1), 21-27, 1992 describes the use of primers designed on the bases of the sequences of the 16S rRNA genes of Mycoplasma pneumoniae and Mycoplasma genitalium in an *in vitro* DNA amplification assay system for the detection of these two mycoplasmas.

Mycoplasma hominis has been implicated as a causative agent of salpingitis, amnionitis, nonspecific vaginitis, and postpartum septic fever. Mycoplasma hominis can be isolated from a significant number of asymptomatic women. It is an aspect of the present invention to describe methods for the design and manufacture of probes specific for the detection of Mycoplasma hominis.

The article by A. Blanchard et al in FEMS Microbiology Letters 81(1991) 37-42 identifies a pair of PCR primers for use in a polymerase chain reaction for the detection of Mycoplasma genitalium, Mycoplasma hominis, Mycoplasma salivarium and Mycoplasma orale.

Ureaplasma urealyticum has been implicated in nongonococcal urethritis, chorioamnionitis, premature delivery, and perinatal morbidity and mortality. Some clinical investigators estimate that the etiological agency of nongonococcal urethritis (NGU) by Ureaplasma urealyticum may approach the same levels as that of Chlamydia trachomatis. As much as 40% of acute NGU may be caused by ureaplasma (Bowie, Urological Clinics of North America, vol. 11, 1984). This translates to approximately 3-4 million United States cases per year. Like Mycoplasma hominis, Ureaplasma urealyticum can be isolated from a significant number of both male and female asymptomatic individuals. There are at least 15 serotypes of Ureaplasma urealyticum. The combination of serotype, numerical prevalence and other factors that contribute to ureaplasma pathogenesis is, at present, totally unknown. It is an aspect of the present invention to describe methods for the design and manufacture of probes specific for the detection of Ureaplasma urealyticum.

The mycoplasmas, such as those described above, are fastidious organisms, requiring complex culture media containing peptone, yeast extract, expensive animal sera, and sterol. Growth is relatively slow and reaches low cell densities compared to most bacteria. In addition, atmospheric conditions for cell growth require the addition of carbon dioxide. For these reasons, many clinical laboratories are unable to perform culture isolation of mycoplasmas, and consequently are left with no real ability to diagnose the presence of these important pathogenic bacteria. Given that mycoplasmas lack cell walls, antibiotics that target the bacterial cell wall, such as penicillin, have no anti-mycoplasma activity. Consequently, it is of importance for a physician to make a diagnosis of the presence of one or more of these bacteria, particularly if the clinical presentation is predictive, and prescribe the appropriate antibiotic.

Ribosomes are of profound importance to all organisms. Ribosomes serve as the only known means of translating genetic information into cellular proteins, the main structural and catalytic elements of life. A clear manifestation of this importance is the observation that all cells have ribosomes.

Bacterial ribosomes contain three distinct RNA molecules which, at least in Escherichia coli, are referred to as 5S, 16S and 23S rRNAs. In eukaryotic organisms, there are four distinct rRNA species, generally referred to as 5S, 18S, 28S, and 5.8S. These names historically are related to the size of the RNA molecules, as determined by their sedimentation rate. In actuality, however, ribosomal RNA molecules vary substantially in size between organisms. Nonetheless, 5S, 16S, and 23S rRNA are commonly used as generic names for the homologous RNA molecules in any bacterium, including the mycoplasmas, and this convention will be continued herein. The probes of the present invention target the 16S and 23S rRNA molecules of the genital mycoplasmas.

Hybridization is a process by which, under appropriate reaction conditions, two partially or completely complementary strands of nucleic acid are allowed to come together in an antiparallel fashion (one oriented 5' to 3', the other 3' to 5') to form a double-stranded nucleic acid with specific and stable hydrogen bonds, following explicit rules pertaining to which nucleic acid bases may pair with one another.

As used herein, probe(s) refer to synthetic or biologically produced nucleic acids (DNA or RNA) which, by design or selection, contain specific nucleotide sequences that allow them to hybridize under hybridization conditions, specifically and preferentially, to target nucleic acid sequences. The term "preferentially" is used in a relative sense, one hybridization reaction product is more stable than another under identical conditions. Under some conditions, a hybridization reaction product may be formed with respect to one target, but not to another potential binding partner. In addition to their hybridization properties, probes also may contain certain constituents that pertain to their proper or optimal functioning under particular assay conditions. For example, probes may be modified to improve their resistance to nuclease degradation (e.g. by end capping), to carry detection ligands (e.g. fluorescein, biotin, etc.), to facilitate detection (e.g. chemiluminescent, fluorescent agents and radioactive agents) or to facilitate their capture onto a solid support (e.g., homopolymer "tails"). Such modifications are elaborations on the basic probe function which is the probe's ability to usefully discriminate between target and non-target organisms in a hybridization assay.

A minimum of ten nucleotides are necessary in order to statistically obtain specificity and form stable hybridization products. A maximum of 250 nucleotides represents an upper limit of sequences in which reaction parameters can be adjusted to determine mismatched sequences and preferential hybridization.

Hybridization conditions are defined by the base composition of the probe/target duplex, as well as by the level and geometry of mispairing between the two nucleic acids. Normal hybridization conditions for nucleic acid of 10 to 250 bases are a temperature of approximately 60°C in the presence of 1.08 M sodium chloride, 60 mM sodium phosphate, and 6 mM ethylenediamine tetraacetic acid (pH of 7.4).

Reaction parameters which are commonly adjusted include concentration and type of tonic species present in the hybridization solution, the types and concentrations of denaturing agents present, and the temperature of hybridization. Generally, as hybridization conditions become more stringent, longer probes are preferred if stable hybrids are to be formed. As a corollary, the stringency of the conditions under which a hybridization is to take place (e.g., based on the type of assay to be performed) will dictate certain characteristics of the preferred probes to be employed. Such relationships are well understood and can be readily manipulated by those skilled in the art.

Kohne et.al. (Biophysical Journal 8:1104-1118, 1968) discuss one method for preparing probes to rRNA sequences. However, Kohne et.al. do not provide the teaching necessary to make probes to detect the three species of genital mycoplasmas.

Pace and Campbell (Journal of Bacteriology 107:543-547, 1971) discuss the homology of ribosomal ribonucleic acids from diverse bacterial species and a hybridization method for quantitating such homology levels. Similarly, Sogin, Sogin and Woese (Journal of Molecular Evolution 1:173-184, 1972) discuss the theoretical and practical aspects of using primary structural characterization of different ribosomal RNA molecules for evaluating phylogenetic relationships. Fox, Pechman and Woese (International Journal of Systematic Bacteriology 27:44-57, 1977) discuss the comparative cataloging of 16S ribosomal RNAs as an approach to prokaryotic systematics. Separately, or together, Kohne et al, Pace and Campbell, Sogin, Sogin and Woese, and Fox, Pechman and Woese fail to provide specific probes useful in assays for detecting the presence and abundance of genital mycoplasmas.

Hogan, et.al. (International Patent Application, Publication Number WO 88/03957) describe five probes for the specific detection of Mycoplasma pneumoniae. However, Hogan et.al. do not describe probes to the specific three genital mycoplasma species.

Woese, Maniloff, Zablen (Proc. Natl. Acad. Sci. USA vol. 77, 1980) examined the partial sequences of selected mycoplasma 16S rRNA, and define the concept of mycoplasma sequence "signature". However, Woese et.al. fail to teach the art of probe design. Woese et.al. do not discuss M. genitalium, M. hominis, or Ureaplasma urealyticum.

Rogers, et.al. (Proc. Natl. Acad. Sci. USA, vol. 82, 1985) discuss sequences of 5S rRNA. However, Rogers et.al. fail to teach the art of probe design. Rogers et.al. do not mention M. genitalium or M. hominis.

Weisburg, et.al. (Jnl. Bacteriology, vol. 171, 1989) discuss 16S rRNA sequences of Ureplasma urealyticum and M. hominis. However, Weisburg et.al. do not discuss or teach mycoplasma probe design.

Hyman, et al. (Hyman, et al., Jnl. Clin. Micro., vol. 25, 1987) and Jensen, et al. (Jensen, et al., Jnl. Clin. Micro. vol 29, 1991) discuss detection of Mycoplasma genitalium using polymerase chain reaction (PCR). Both papers fail to teach non-PCR assays with respect to the utility of the sequences of mycoplasma rRNA or rDNA.

### Summary of the Invention

The present invention features nucleic acid compositions and composition sets, and methods for their use for the specific detection or identification of Mycoplasma hominis, Ureaplasma urealyticum, and Mycoplasma genitalium. The present invention provides a nucleic acid having approximately 10 to 250 nucleotides capable of hybridizing preferentially to rRNA or rDNA of pathogenic mycoplasma bacteria selected from Mycoplasma hominis, Ureaplasma urealyticum and Mycoplasma genitalium which are associated with human urinary tract and genital areas wherein said nucleic acid hybridizes to at least one region of rRNA or rDNA selected from regions consisting of:
positions 50 to 100, 425 to 485 or 1100 to 1150 of Mycoplasma hominis 16S rRNA;
positions 50 to 100, 150 to 250, 425 to 485, 800 to 850, 1090 to 1160 or 1220 to 1260 of Ureaplasma urealyticum 16S rRNA;
positions 1110 to 1160 of Mycoplasma genitalium 16S rRNA; and
positions 260 to 330, 1590 to 1630 or 1850 to 1900 of Mycoplasma genitalium 23S rRNA. The nucleic acid composition is useful for detecting Mycoplasma hominis, Ureaplasma urealyticum and Mycoplasma genitalium. As such numerical designations are nucleotide positions counted from the 5' end of the RNA molecule, a convention known to those skilled in the art.

Preferably, the nucleic acid composition is complementary to at least 90% of a sequence comprising any ten consecutive nucleotides within a nucleic acid sequence selected from the group consisting of probes 2262, 2256, 2246, 2218, 2271, 2220, 2259, 2227, 2335, 2337, 2334, 2336, 2191, 2228, and 2260.

A further embodiment of the present invention includes a nucleic acid which is homologous to at least 90% of a sequence comprising any ten consecutive nucleotides within the sequences selected from the group consisting of probes 2262, 2256, 2246, 2218, 2271, 2220, 2259, 2227, 2335, 2337, 2334, 2336, 2191, 2228, and 2260.

One embodiment of the present invention features as an article of manufacture a set of at least two nucleic acid compositions. Each nucleic acid composition having approximately 10 to 250 nucleotides capable of hybridizing preferentially to the rRNA or rDNA of Mycoplasma hominis, Ureaplasma urealyticum and Mycoplasma genitalium according to the invention. Each nucleic acid is complementary to or homologous with at least 90% of a sequence comprising any ten consecutive nucleotides within the sequences selected from the group defined by probes consisting of 2262, 2256, 2246, 2218, 2271, 2220, 2259, 2227, 2335, 2337, 2334, 2336, 2191, 2228, and 2260.

Probe sets suited for detecting Ureaplama urealyticum include probe 2262 and probe 2256; probe 2246 and probe 2218; probe 2259 and probe 2271; probe 2271 and probe 2220; and their complements.

Probe sets that are suited for detecting Mycoplasma hominis include probe 2191 and probe 2228; probe 2228 and probe 2260; probe 2191 and probe 2260; and their complements.

Probe sets which are suitable for detecting Mycoplasma genitalium include probe 2227 and probe 2219; probe 2335 and probe 2337; probe 2335 and probe 2334; probe 2334 and probe 2336; and their complements. The probe 2227 and probe 2219 are capable of hybridization to the rRNA of the 16S ribosomal subunit. The probe sets probe 2335 and probe 2337; probe 2335 and probe 2334; probe 2334 and probe 2336 are capable of hybridizing to the 23S ribosomal subunit.

Embodiments of the present invention are also directed to methods for detecting Mycoplasma hominis, Ureaplasma urealyticum, and Mycoplasma genitalium. The method includes contacting a sample potentially containing the microorganisms with at least one nucleic acid composition having 10 to 250 nucleotides capable of hybridizing preferentially to rRNA or rDNA of Mycoplasma hominis, Ureaplasma urealyticum, and Mycoplasma genitalium, wherein said nucleic acid hybridizes to at least one region of rRNA or rDNA selected from regions consisting of:
positions 50 to 100, 425 to 485 or 1100 to 1150 of Mycoplasma hominis 16S rRNA:
positions 50 to 100, 150 to 250, 425 to 485, 800 to 850, 1090 to 1160 or 1220 to 1260 of Ureaplasma urealyticum 16S rRNA;
positions 1110 to 1160 of Mycoplasma genitalium 16S rRNA; and
positions 260 to 330, 1590 to 1630 or 1850 to 1900 of Mycoplasma genitalium 23S rRNA. The base sequences, under conditions that allow the nucleic acid composition to hybridize, hybridize preferentially to the rRNA or rDNA of the mycoplasma organism. Upon imposition of hybridization conditions on the sample, a hybridization product is formed in the presence of target. The detection of the hybridization product is an indication of the presence of the mycoplasma.

According to an embodiment of the invention, the method features nucleic acid compositions which are complementary to or homologous with at least 90% of a 10 nucleotide sequence within the sequences selected from the group defined by probes consisting of 2219, 2262, 2256, 2246, 2218, 2271, 2220, 2259, 2227, 2335, 2337, 2334, 2336, 2191, 2228 and 2260.

Embodiments of the present invention also feature a method employing a first nucleic acid composition and a second nucleic acid composition each of which is a different sequence, and each of which is complementary to or homologous with at least 90% of 10 nucleic acid sequences within the group of sequences defined by probes consisting of 2219, 2262, 2256, 2246, 2218, 2271, 2220, 2259, 2227, 2335, 2337, 2334, 2336, 2191, 2228 and 2260. Preferably the sequences of each set are probe 2262 and probe 2256; probe 2246 and probe 2218; probe 2259 and probe 2271; probe 2271 and probe 2220; probe 2191 and probe 2228; probe 2228 and probe 2260; probe 2191 and probe 2260; probe 2227 and probe 2219; probe 2335 and probe 2337; probe 2335 and probe 2334; and probe 2334 and probe 2336.

Individuals skilled in the art will recognize that the probe compositions of the present invention can be packaged with suitable instructions for their use as kits for detecting the presence of one or more of the organisms Mycoplasma hominis, Ureaplasma urealyticum and Mycoplasma genitalium.

The nucleic acid compositions and the methods of the present invention provide the basis for nucleic acid hybridization assay for the specific detection of three of the presumptive etiological agents of nongonococcal urethritis, septic abortion, pelvic inflammatory disease, postpartum fever, in clinical samples such as genital swabs, genital lavage, sputum, throat swabs, blood, urine, cerebrospinal fluid, skin, biopsy, saliva, synovial fluid, bronchial wash, bronchial lavage, or other tissue or fluid samples from human patients or veterinary subjects. The nucleic acid compositions of the present invention also form the basis for confirmation of the presence of the genital mycoplasmas in liquid or semi-solid in vitro culture.

Embodiments of the present invention feature nucleic acid compositions capable of hybridizing to rRNA. Ribosomal RNAs constitute a significant component of cellular mass. Although estimates of cellular ribosome content vary, actively growing bacterial cells may contain upwards of 10,000 ribosomes per cell, and therefore 10,000 copies of each of the rRNAs present in a 1:1:1 stoichiometry in ribosomes. In contrast, other potential cellular target molecules such as genes or RNA transcripts thereof, are less ideal since they are present in much lower abundance. However, due to the close similarity of many organisms with which you may wish to distinguish mycoplasma species from, there can be no assurance that nucleic acid compositions can indeed be formulated which hybridize preferentially to the rRNA and rDNA of Mycoplasma hominis, Ureaplasma urealyticum and Mycoplasma genitalium.

The discovery that probes could be generated with the extraordinary inclusivity and exclusivity characteristics of those of the present invention with respect to the detection of genital mycoplasma isolates, without necessarily incurring cross-reactivity between these species, or toward other bacterial taxa, except as documented, was unpredictable and unexpected.

### Brief Description of the Tables and Figure

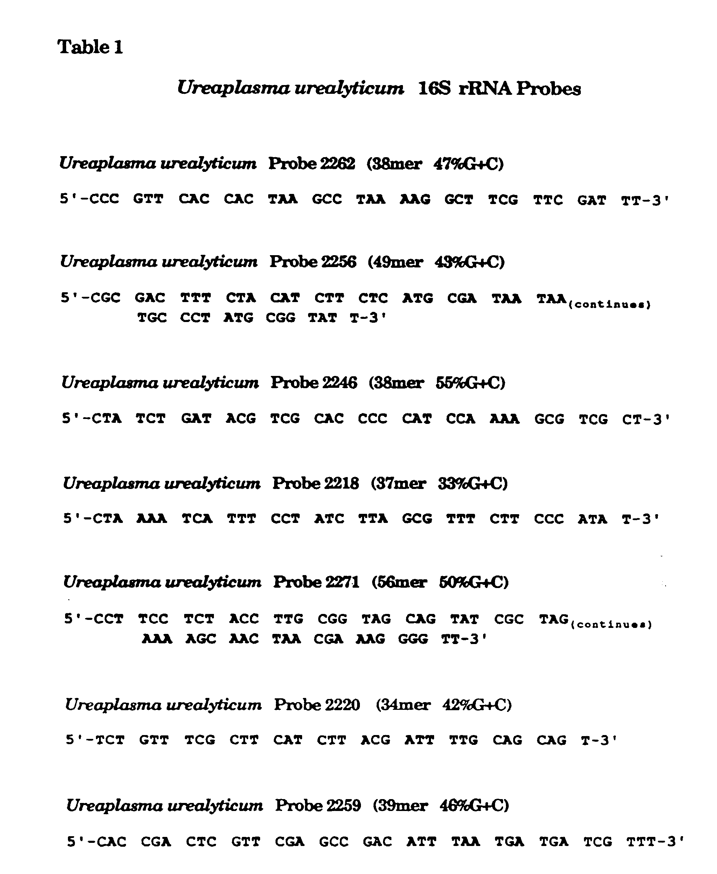

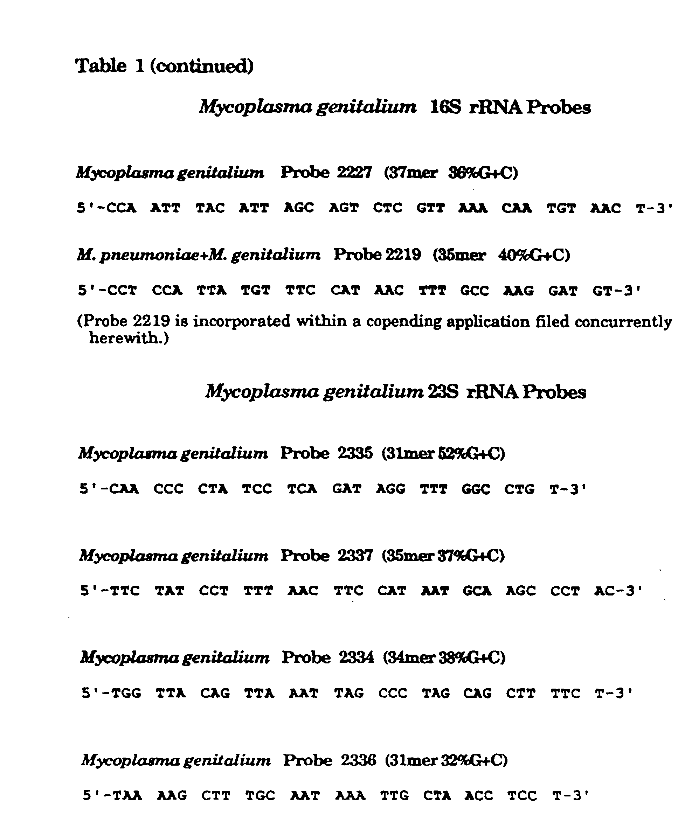

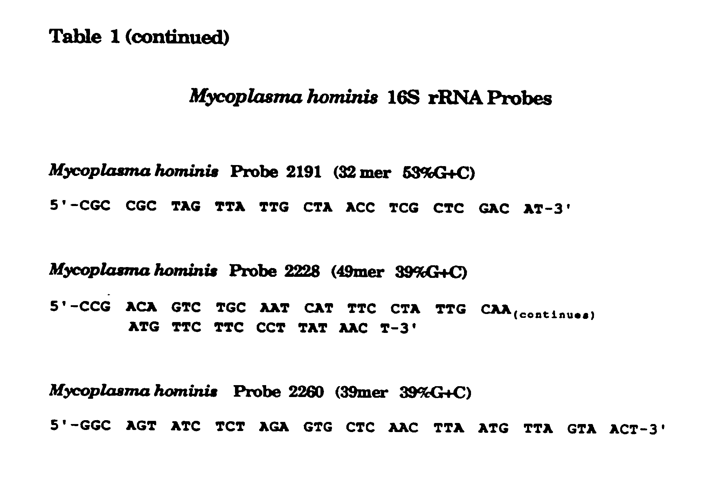

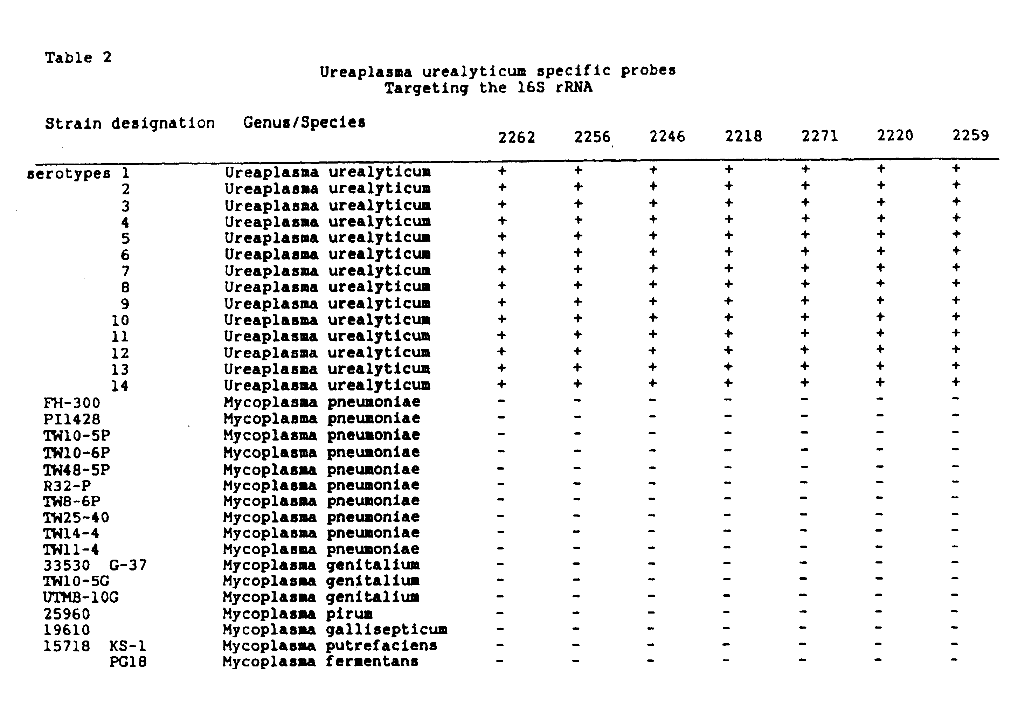

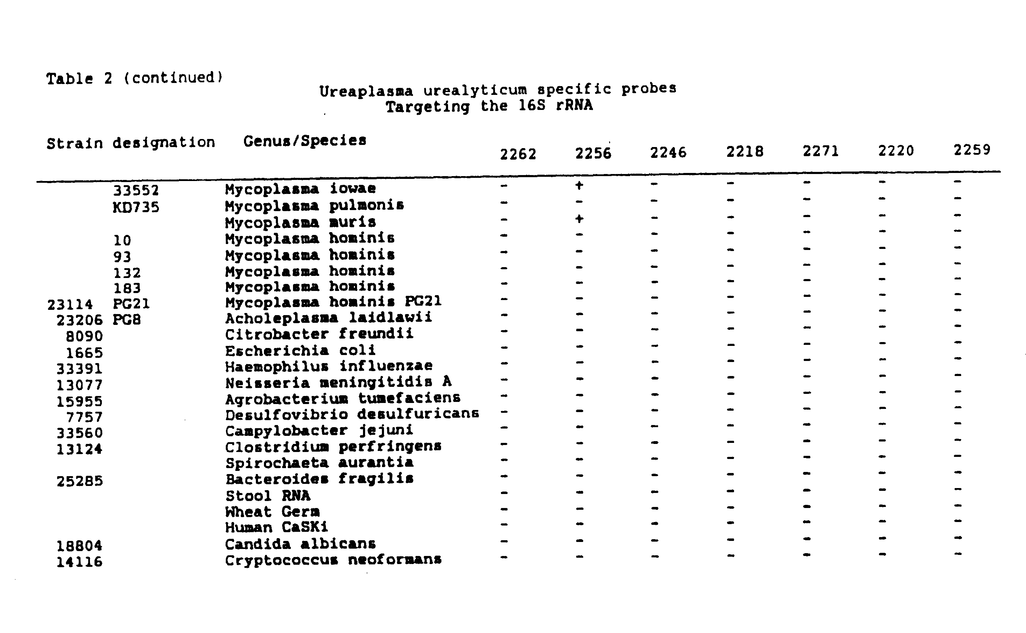

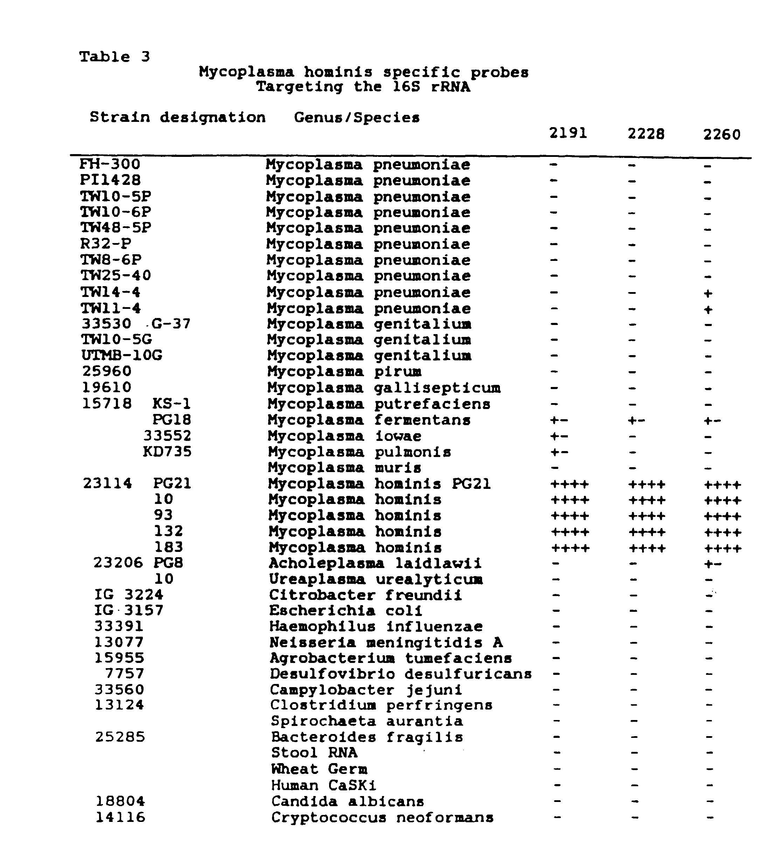

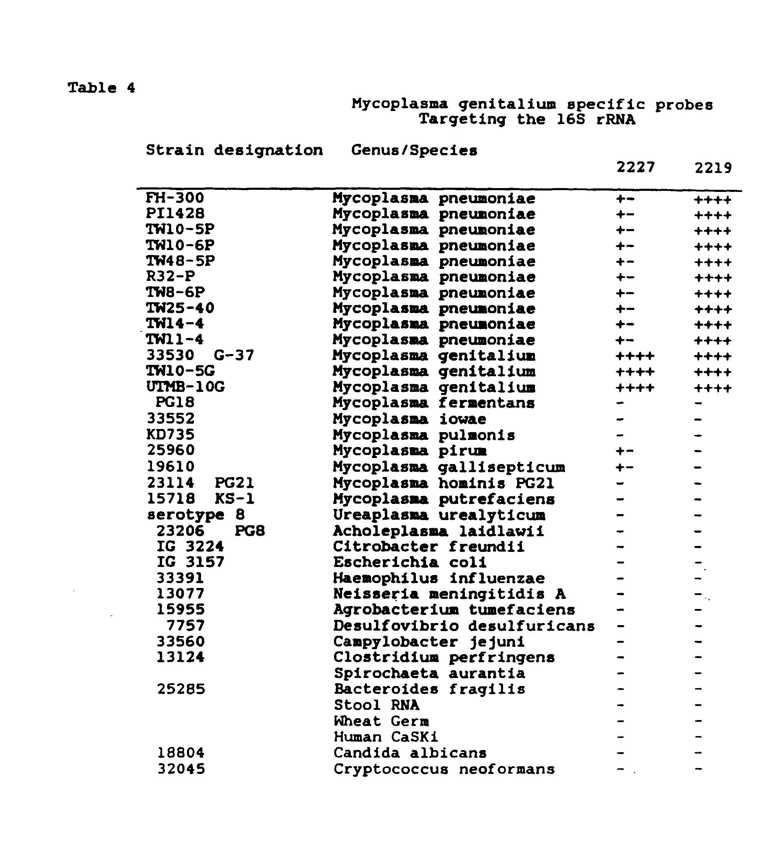

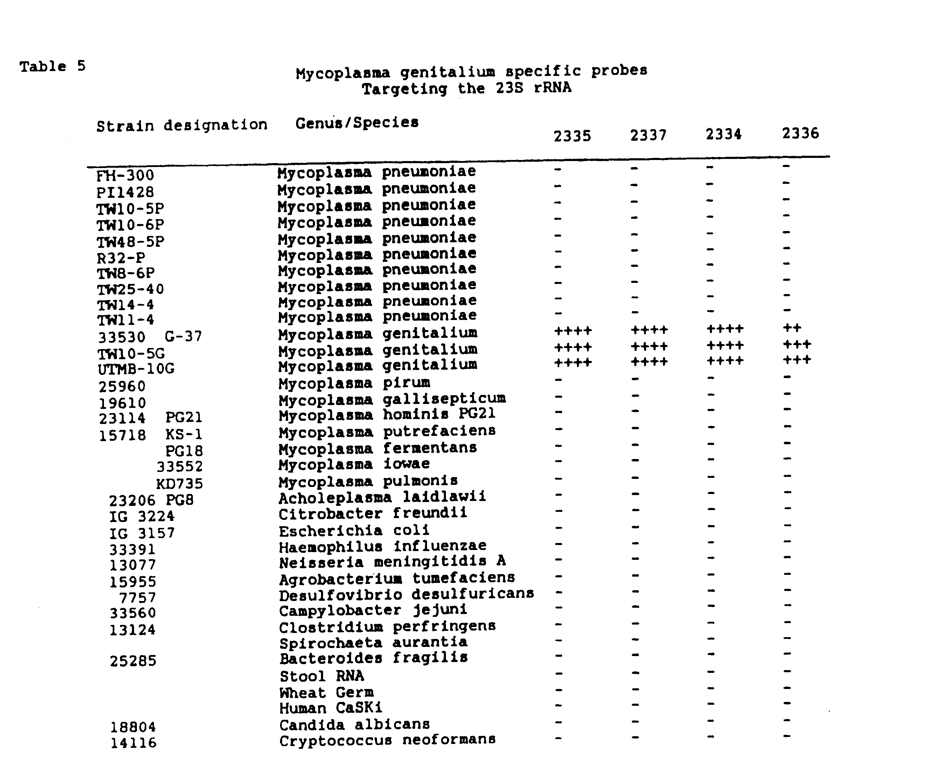

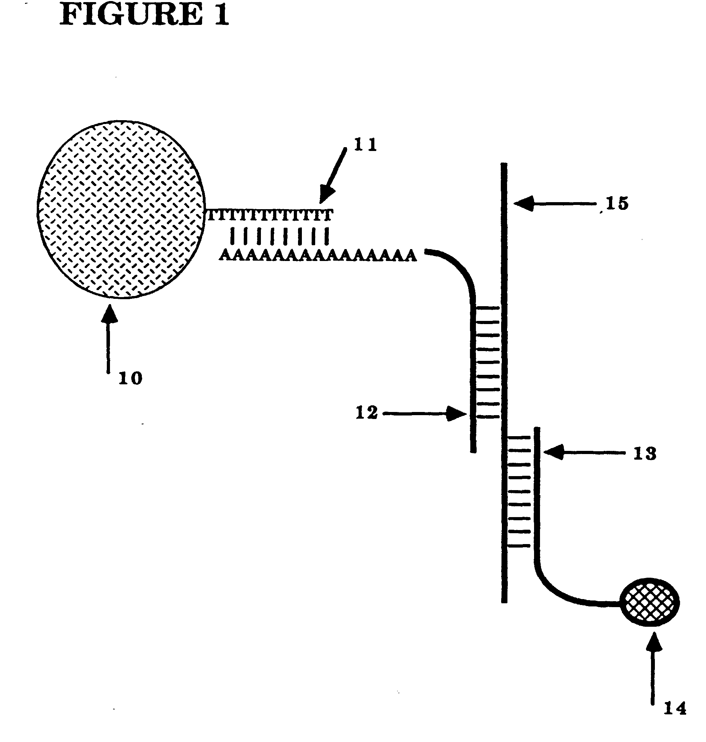

A further understanding of the principles and aspects of the present invention may be made by reference to the tables and Figure 1 described briefly below:

Table 1 describes the physical structure of the probes.

Tables 2, 3, 4, and 5 display the hybridization behavior of the probes toward a panel of clinically and environmentally representative mycoplasma species.

Figure 1 is a schematic representation of a dual probe capture/detector assay.

### Detailed Description of the Invention and Best Mode

Preferred embodiments of the present invention will be discussed with respect to nucleic acid compositions having particular utility as probes for Mycoplasma hominis, Ureaplasma urealyticum and Mycoplasma genitalium. The detailed discussion will feature probe development strategy, dot blot analysis, sandwich hybridization assays, instrument hybridization and amplification.

### I. Probe Development Strategy

The first step taken in the development of the probes of the present invention involved identification of regions of the 16S rRNA and 23S rRNA which potentially could serve as target sites for specific nucleic acid probes with the desired sensitivity. To obtain the desired sensitivity a search began for probe targets unique to Mycoplasma hominis, Ureaplasma urealyticum, and Mycoplasma genitalium.

Precise alignments of mycoplasma 16S and 23S rRNA sequences were developed. The essentially complete 16S rRNA and 23S rRNA sequences from Mycoplasma genitalium were determined. The 16S rRNA sequences from Ureaplasma urealyticum and Mycoplasma hominis are those of Weisburg, et.al. (Journal of Bacteriology, vol. 171, 1989) and are available through GENBANK accession numbers M23935 and M24473.

The nucleotide sequences from M. genitalium were determined by standard laboratory protocols well known to those skilled in the art of probe design. By way of example, the sequences can be determined from plasmid clones which were generated by ligating polymerase chain reaction products from 16S rDNA and 23S rDNA into restriction digested plasmid vectors.

The mycoplasma sequences were aligned with homologous sequences of other mycoplasma and nonmycoplasma ribosomal RNA sequences in order to identify regions of interesting variation. The regions of interesting variation are identified with each probe construct of Table 1.

In addition to the consideration of the target sequences of M. hominis, M. genitalium, and U. urealyticum, the exclusion from hybridization of other mycoplasmas, and other genital and pathogenic organisms was considered. Probe regions were compared to 16S rRNA sequences of the following organisms (GenBank accession numbers shown in Weisburg, et.al., Jnl. Bacteriology, vol. 171, 1989): M. agalactiae, M. arginini, M. arthritidis, M. bovigenitalium, M. californicum, M. capricolum, M. ellychniae, M. melaleucum, M. fermentans, M. gallisepticum, M. hyopneumoniae, M. hyorhinis, M. iowae, M. lipophilum, M. mobile, M. muris, M. mycoides, M. neurolyticum, M. orale, M. pirum, M. pneumoniae, M. pulmonis, M. putrefaciens, M. salivarium, M. sualvi, ten Spiroplasma species' sequences, four Acholeplasma species' sequences, four Anaeroplasma species' sequences, and one Asteroleplasma sequence.

For consideration of other microorganisms capable of causing similar types of genital morbidity, rRNA sequences from several Candida yeasts, Neisseria gonorrhoeae, Chlamydia trachomatis, Treponema pallidum, and numerous other species were utilized, the majority of which are available from sequence compilation such as GenBank. Individuals skilled in the art will recognize that sequences of interest as probes can be compared to other organisms as well.

Sixteen probes--designed, synthesized, and tested--are described in Table 1. Seven probes show interesting specificity toward Ureaplasma urealyticum 16S rRNA. Three probes show interesting specificity toward Mycoplasma hominis 16S rRNA. One probe shows interesting specificity toward Mycoplasma genitalium 16S rRNA. And, four probes show interesting specificity toward Mycoplasma genitalium 23S rRNA.

### Description of the Probes

The probe selection strategy yielded sixteen nucleic acid compositions useful for hybridization to the genital mycoplasmas in clinical or other samples. The specific behavior of the probes in one format is documented in Tables 2, 3, 4, and 5.

Referring now to said Tables, the three representative strains of M. genitalium represent a selection from the National Institute of Allergy and Infectious Disease's Mycoplasma Section. The five representative Mycoplasma hominis strains and all 15 serovars of Ureaplasma urealyticum are a representative collection from the University of Alabama at Birmingham mycoplasma collection. Additional species of bacteria and a few fungi were added to the panel in order to represent the breadth of known bacterial taxa.

Specific strains of species are represented by the application of purified, denatured RNA to a membrane and probe hybridization thereto. Probes were synthesized by standard methods and chemistries well known to those skilled in the art, ³²Phosphorous labelled, hybridized to panels under standard conditions, at 60°C and autoradiographically evaluated.

In Table 2, hybridization is expressed as either "+" or "-". Positive hybridization is evaluated based on autoradiographic detection of a signal after three hours exposure. The probes in this panel were evaluated in this manner because cultures of Ureaplasma urealyticum seldom achieve cell density greater than 1 million cells per milliliter. In order to normalize the signals generated by hybridization of the ureaplasma probes to the Ureaplasma urealyticum targets, hybridization signal was compared to that of a probe known to hybridize to all bacterial species (co-pending USSN 359,158; Probe 1660). If signals from Probe 1660 were very weak, the candidate ureaplasma probe's signal was "normalized" accordingly. All of the Ureaplasma urealyticum "+" designations would accordingly be considered "+++" or "++++" if the amount of nucleic acid spotted for each of the strains were adjusted so as to give equal signals to the control probe, Probe 1660.

In Tables 3, 4, and 5 "++++" represents strongest hybridization signal after three hours exposure, with slightly lighter signal represented by "+++", diminishing to "++", then "+". "+-" is virtually absent, and "-" is indicative of no hybridization of probe to target.

The specific behaviors of the aforementioned probes are dependent to a significant extent on the assay format in which they are employed. Conversely, the assay format will dictate certain of the optimal features of particular probes. The "essence" of the probes of the invention is not to be construed as restricted to the specific string of nucleotides in the named probes. For example, the length of these particular oligonucleotides was optimized for use in the dot blot assay and sandwich assays. It is well known to those skilled in the art that optimal probe length will be a function of the stringency of the hybridization conditions chosen and hence the length of the probes disclosed herein may be altered accordingly. Generally, in order to create stability and obtain sufficient exclusivity and inclusivity, probe sequences comprise at least 10 nucleotides. Probe sequences rarely exceed 250 nucleotides. The longer nucleic acids are more difficult to control to obtain necessary exclusivity and inclusivity.

In considering sets comprised of more than one probe, it is desirable that all probes behave in a compatible manner in any particular format in which they are employed. Thus, the exact length of a particular probe will to a certain extent reflect its specific intended use.

The nucleic acids of the present invention circumscribe sequences which can be employed as oligonucleotide probes, or could be incorporated into larger polynucleotides of either ribonucleic acid or deoxyribonucleic acid. Sequences complementary to the probe sequences described herein can be used as probes to rRNA genes. The preferred probe sequences or their complements can be employed as chain elongation initiators for polymerase chain reaction, sequencing or other applications.

The physical description of the probes can be ascertained with reference to the incorporated Tables 1 and 2. The abbreviations for nucleotides--G, A, C, and T--as well as polarity indicated by 5' and 3' are standard designations well known to one skilled in the art.

The experimental specificity of the preferred probes, as described in Example 1 is summarized in Tables 2, 3, 4, and 5. The specificity is empirical and as stated previously dependent on the format in which they are employed.

### Examples

### Example 1. Dot-Blot Analysis of Probe Hybridization Behavior

Dot-blot analysis, in accordance with well known procedures, involves immobilizing a nucleic acid or a population of nucleic acids on a filter such as nitrocellulose, nylon, or other derivatized membranes which can readily be obtained commercially, specifically for this purpose. Either DNA or RNA can be easily immobilized on such a filter and subsequently can be probed or tested for hybridization under any of a variety of conditions (i.e., stringencies) with nucleotide sequences or probes of interest. Under stringent conditions, probes whose nucleotide sequences have greater complementarity to the target will exhibit a higher level of hybridization than probes containing less complementarity.

Nucleic acid sequences of the present invention were tested in a dot-blot format. One hundred nanograms of RNA, except Ureaplasma, as indicated, purified by phenol extraction and ethanol precipitation was denatured and spotted on a nylon membrane. Probes were isotopically labelled with the addition of a ³²Phosphorous moiety to the 5' end of the oligonucleotide. Hybridization of probes occurred, at a temperature of 60°C in the presence of 1.08 M sodium chloride, 60 mM sodium phosphate, and 6 mM ethylenediamine tetraacetic acid, pH 7.4. Unhybridized probe was removed by washing at a salt concentration one-third of the hybridization condition. The filters were exposed to X-ray film and the intensity of hybridization signals was evaluated after three hours of exposure.

Tables 2, 3, 4, and 5 summarize the behavior of the probes as employed in the present example, and document the specificity summarized above.

### Example 2. Dual Probe Hybridization

One embodiment of the present invention includes the use of probes in a "sandwich" hybridization scheme. Turning now to Figure 1, the sandwich hybridization scheme employs a capture probe 12 and detector probe 13. The capture probe 12 is a bifunctional polynucleotide manufactured by adding a homopolymeric 3' tail to probe sequences with high target specificity. The tail would, in turn, hybridize to the complimentary homopolymer 11 on a solid surface 10, such as a glass bead or a filter disc. Hybridization of the capture probe 12 to its target 15, in this case mycoplasma rRNA, forms a target-probe complex with the solid support 10. The detector probe 13, is capable of binding to target with some degree of specificity, would be part of a detection scheme relying on radioactivity, fluorescence, chemiluminescence, color, and the like. Detector probe 13 has a detection moiety 14 which would report the presence of the entire hybridization complex. The detector probe 13 could potentially be incorporated as an RNA sequence into an amplifiable Q-beta midivariant as described by Kramer and Lizardi (Nature, vol. 339, 1989).

### Example 3. Clinical diagnosis of genital mycoplasma infection

A clinical sample, such as a swab, urine, or lavage is processed so as to liberate the total nucleic acid acid content. The sample, containing disrupted mycoplasmas is incubated in the presence of capture probe, detector probe, and magnetic particle beads which have been derivatized with oligo-Thymidine--as in Example 2--in a chaotropic buffer such as guanidinium isothiocyanate.

If target molecules, Mycoplasma genitalium 16S or 23S rRNA, Ureaplasma urealyticum 16S rRNA, or Mycoplasma hominis 16S rRNA (depending on which probe sets are employed) are present, a Bead + Capture Probe + Target + Detector Probe hybridization complex is formed, as in Figure 1. The presence of a magnet near the bottom of the reaction tube will cause the magnetic particle now carrying the hybridization complex to adhere to the side of the tube enabling removal of the sample matrix, unbound probe, etc. Repeated rehydration and denaturation of the bead+probe+target complex would enable significant background reduction (as described in USSN 922,155, Collins, 1986). In this example, final detection could entail spotting the beads on membrane and assaying by autoradiography. Alternatively, the detector probe could be an amplifiable midivariant probe as described in Example 2.

For this particular assay, the following capture and detector probes are examples of preferred pairs:
(1) Ureaplasma urealyticum 16S rRNA
   Probe 2262 + Probe 2256
   Probe 2246 + Probe 2218
   Probe 2259 + Probe 2271
   Probe 2271 + Probe 2220
(2) Mycoplasma hominis 16S rRNA
   Probe 2191 + Probe 2228
   Probe 2228 + Probe 2260
   Probe 2191 + Probe 2260
(3) Mycoplasma genitalium 16S rRNA
   Probe 2227 + Probe 2219
(4) Mycoplasma genitalium 23S rRNA
   Probe 2335 + Probe 2337
   Probe 2335 + Probe 2334
   Probe 2334 + Probe 2336

### Example 4. Clinical diagnosis of M. hominis, M. genitalium, or U. urealyticum infection from human sample employing polymerase chain reaction amplification of mycoplasma rDNA

Sample processing is designed so as to yield DNA. One of the probes described herein is used in conjunction with the antiparallel complement of one of the probes described herein to enzymatically amplify a segment of M. genitalium, M. hominis, U. urealyticum encoding mycoplasma rRNA in a polymerase chain reaction. Resultant material can then be assayed in a "sandwich" hybridization (Example 2) with any of the probes described herein. The polymerase chain reaction can, itself, be made either highly specific by employing probe/primers described herein, or the reaction can be made more general using probes such as those described in co-pending USSN 359,158, and then identifying the amplification product as a genital mycoplasma as described in Example 2.

### Example 5. In situ hybridization as a cytological stain

The probes of the present invention could also be employed as cytological staining reagents. For example, a genital swab is applied to a microscope slide. After appropriate fixation and lysis, hybridization of probes is carried out in situ. In this example, mycoplasmas could be visualized in a specimen by fluorescently labelling Probes 2227, 2260, and 2271, and examining the slide using a fluorescent microscope, looking for small fluorescent bodies.

### Example 6. Confirmation of presumptive mycoplasma species identification following culture

Following a standard cultivation step for Mycoplasma hominis, M. genitalium, or Ureaplasma urealyticum, for example on H-agar plates, SP-6 broth, 10B broth, or A8 agar, a colony or liquid culture is tested for the presence of one of the genital mycoplasmas by employing sandwich hybridization assays as described in Examples 2 and 3. A pure culture is not necessary.

Thus, the present invention features nucleic acid compositions which are suitable for use in detecting the presence of Mycoplasma hominis, Ureaplasma urealyticum and Mycoplasma genitalium. Individuals skilled in the art will readily recognize that probes, reagents and compositions necessary to test a sample for the presence of one of these organisms may be packaged in suitable containment vessels assembled into a kit. Such probes, reagents and compositions of the kit would be used to test a sample for one or more of the organisms specified above.

## Claims

1. A nucleic acid having approximately 10 to 250 nucleotides capable of hybridizing preferentially to rRNA or rDNA of pathogenic mycoplasma bacteria selected from Mycoplasma hominis, Ureaplasma urealyticum and Mycoplasma genitalium which are associated with human urinary tract and genital area wherein said nucleic acid hybridizes to at least one region of rRNA or rDNA selected from regions consisting of:
positions 50 to 100, 425 to 485 or 1100 to 1150 of Mycoplasma hominis 16S rRNA;
positions 50 to 100, 150 to 250, 425 to 485, 800 to 850, 1090 to 1160 or 1220 to 1260 of Ureaplasma urealyticum 16S rRNA;
positions 1110 to 1160 of Mycoplasma genitalium 16S rRNA; and
positions 260 to 330, 1590 to 1630 or 1850 to 1900 of Mycoplasma genitalium 23S rRNA.

2. The nucleic acid of claim 1, wherein said nucleic acid is complementary to at least 90% of a sequence comprising any ten consecutive nucleotides with a nucleic acid sequence selected from the group consisting of sequences 2262, 2256, 2246, 2218, 2271, 2220, 2259, 2227, 2335, 2337, 2334, 2336, 2191, 2228, and 2260.

3. The nucleic acid of claim 1, wherein said nucleic acid is homologous to at least 90% of a sequence comprising any ten consecutive nucleotides selected from the group of sequences defined by probes consisting of probes 2262, 2256, 2246, 2218, 2271, 2220, 2259, 2227, 2335, 2337, 2334, 2336, 2191, 2228 and 2260.

4. A set of at least two nucleic acid compositions, each nucleic acid composition having approximately 10 to 250 nucleotides of different sequence compositions, capable of preferentially hybridizing to rRNA or rDNA of pathogenic mycoplasma bacteria selected from Mycoplasma hominis, Ureaplasma urealyticum and Mycoplasma genitalium which are associated with human urinary tract and genital areas, wherein each nucleic acid is complementary to or homologous to at least 90% of a sequence comprising any 10 consecutive nucleotides selected from the group of sequences defined by probes 2262, 2256, 2246, 2218, 2271, 2220, 2259, 2227, 2335, 2337, 2334, 2336, 2191, 2228 and 2260, and said remaining nucleic acid is selected from the remaining sequence.

5. A set of nucleic acids of claim 4 wherein said set is selected from the group of nucleic composition sets defined by probes:
probe 2262 and probe 2256;
probe 2246 and probe 2218;
probe 2259 and probe 2271;
probe 2271 and probe 2220; and their complements, wherein said set is capable of detecting Ureaplasma urealyticum.

6. A set of nucleic acids of claim 4 wherein said set is selected from the group of nucleic acid composition sets defined by probes:
probe 2191 and probe 2228;
probe 2228 and probe 2260;
probe 2191 and probe 2260; and their complements. wherein said set is capable of detecting Mycoplasma hominis.

7. A set of nucleic acids of claim 4 wherein said set is selected from the group of nucleic acid composition sets defined by probe 2227 and probe 2219, and their complements, wherein said set is capable of detecting Mycoplasma genitalium.

8. A set of nucleic acids of claim 4 wherein said set is selected from the group of nucleic acid composition sets defined by probes:
probe 2335 and probe 2337;
probe 2335 and probe 2334;
probe 2334 and probe 2336 and their complements, wherein said set is capable of detecting Mycoplasma genitalium.

9. A method for detecting Mycoplasma hominis, Ureaplasma, urealyticum, or Mycoplasma genitalium in a sample comprising:
a) contacting said sample with at least one nucleic acid composition having 10 to 250 nucleotides which, under conditions that allow said nucleic acid composition to hybridize preferentially to rRNA or rDNA of said mycoplasma organism, wherein said nucleic acid hybridizes to at least one region of rRNA or rDNA selected from regions consisting of:
positions 50 to 100, 425 to 485 or 1100 to 1150 of Mycoplasma hominis 16S rRNA;
positions 50 to 100, 150 to 250, 425 to 485, 800 to 850, 1090 to 1160 or 1220 to 1260 of Ureaplasma urealyticum 16S rRNA;
positions 1110 to 1160 of Mycoplasma genitalium 16S rRNA; and
positions 260 to 330, 1590 to 1630 or 1850 to 1900 of Mycoplasma genitalium 23S rRNA,
b) imposing hybridization conditions on said sample to form a hybridization product in the presence of target; and,
c) detecting said hybridization product as an indication of the presence of said mycoplasma.

10. The method of claim 9 wherein said nucleic acid composition of said contacting step is complementary to or homologous with at least 90% of any 10 consecutive nucleotides selected from the group of sequences defined by probes consisting of 2219, 2262, 2256, 2246, 2218, 2271, 2220, 2259, 2227, 2335, 2337, 2334, 2336, 2191, 2228 and 2260.

11. The method of claim 9 wherein said contacting step employs a first nucleic acid composition and a second nucleic acid composition, wherein said first and second nucleic acids have different sequences, which are complementary to or homologous with at least 90% of any 10 consecutive nucleotides selected from the group of sequences defined by probes consisting of 2219, 2262, 2256, 2246, 2218, 2271, 2220, 2259, 2227, 2335, 2337, 2334, 2336, 2191, 2228 and 2260.

12. The method of claim 11 wherein said first nucleic acid composition and said second nucleic acid composition are sets selected from the group of sets defined by probes consisting of:
probe 2262 and probe 2256;
probe 2246 and probe 2218;
probe 2259 and probe 2271;
probe 2271 and probe 2220;
probe 2191 and probe 2228;
probe 2228 and probe 2260;
probe 2191 and probe 2260;
probe 2227 and probe 2219;
probe 2335 and probe 2337;
probe 2335 and probe 2334; and
probe 2334 and probe 2336.

13. A kit for detecting the presence of one or more organisms selected from Mycoplasma hominis, Ureaplasma urealyticum and Mycoplasma genitalium which comprises a nucleic acid having 10 to 250 nucleotides capable of hybridizing preferentially to rRNA or rDNA of said organisms wherein said nucleic acid hybridizes to at least one region of rRNA or rDNA selected from regions consisting of:
positions 50 to 100, 425 to 485 or 1100 to 1150 of Mycoplasma hominis 16S rRNA;
positions 50 to 100, 150 to 250, 425 to 485, 800 to 850, 1090 to 1160 or 1220 to 1260 of Ureaplasma urealyticum 16S rRNA;
positions 1110 to 1160 of Mycoplasma genitalium 16S rRNA; and
positions 260 to 330, 1590 to 1630 or 1850 to 1900 of Mycoplasma genitalium 23S rRNA.

## Patentansprüche

1. Nukleinsäure mit etwa 10 bis 250 Nukleotiden, die in der Lage sind, vorzugsweise an rRNA oder rDNA pathogener Mycoplasmen-Bakterien, ausgewählt aus Mycoplasa hominis, Ureaplasma urealyticum und Mycoplasma genitalium, die mit dem menschlichen Harnapparat und Genitalbereichen assoziiert sind, zu hybridisieren, dadurch **gekennzeichnet**, daß die Nukleinsäure an wenigstens eine rRNA- oder rDNA-Region, ausgewählt aus Regionen bestehend aus:
Positionen 50 bis 100, 425 bis 485 oder 1100 bis 1150 der Mycoplasma hominis 16S rRNA;
Positionen 50 bis 100, 150 bis 250, 425 bis 485, 800 bis 850, 1090 bis 1160 oder 1220 bis 1260 der Ureaplasma urealyticum 16S rRNA;
Positionen 1110 bis 1160 der Mycoplasma genitalium 16S rRNA; und
Positionen 260 bis 330, 1590 bis 1630 oder 1850 bis 1900 der Mycoplasma genitalium 23S rRNA hybridisiert.

2. Nukleinsäure nach Anspruch 1, dadurch **gekennzeichnet** daß die Nukleinsäure zu wenigstens 90% einer Sequenz komplementär ist, die jegliche zehn aufeinanderfolgende Nukleotide mit einer Nukleinsäuresequenz, ausgewählt aus der Gruppe bestehend aus den Sequenzen 2262, 2256, 2246, 2218, 2271, 2220, 2259, 2227, 2335, 2337, 2334, 2336, 2191, 2228 und 2260, umfaßt.

3. Nukleinsäure nach Anspruch 1, dadurch **gekennzeichnet** daß die Nukleinsäure zu wenigstens 90% einer Sequenz homolog ist, die jegliche zehn aufeinanderfolgende Nukleotide, ausgewählt aus der Gruppe von Sequenzen, die durch Sonden, bestehend aus den Sonden 2262, 2256, 2246, 2218, 2271, 2220, 2259, 2227, 2335, 2337, 2334, 2336, 2191, 2228 und 2260, definiert werden, umfaßt.

4. Satz von wenigstens zwei Nukleinsäurezusammensetzungen, wobei jede Nukleinsäurezusammensetzung wenigstens 10 bis 250 Nukleotide verschiedener Sequenzzusammensetzungen hat und in der Lage ist, vorzugsweise an rRNA oder rDNA pathogener Mycoplasmen-Bakterien, ausgewählt aus Mycoplasma hominis, Ureaplasma urealyticum und Mycoplasma genitalium, die mit dem menschlichen Harnapparat und Genitalbereichen assoziiert sind, zu hybridisieren, dadurch **gekennzeichnet** daß jede Nukleinsäure komplementär oder homolog zu wenigstens 90% einer Sequenz ist, die jegliche zehn aufeinanderfolgenden Nukleotide, ausgewählt aus der Gruppe von Sequenzen, die durch die Sonden 2262, 2256, 2246, 2218, 2271, 2220, 2259, 2227, 2335, 2337, 2334, 2336, 2191, 2228 und 2260 definiert werden, umfaßt und daß die übrige Nukleinsäure aus der übrigen Sequenz ausgewählt ist.

5. Satz von Nukleinsäuren nach Anspruch 4, dadurch **gekennzeichnet,** daß der Satz aus der Gruppe von Nukleinsäurezusammensetzungs-Sätzen, die durch die folgenden Sonden definiert sind:
Sonde 2262 und Sonde 2256;
Sonde 2246 und Sonde 2218;
Sonde 2259 und Sonde 2271;
Sonde 2271 und Sonde 2220; und ihre komplementären Sonden ausgewählt ist, wobei jeder Satz in der Lage ist, Ureaplasma urealyticum nachzuweisen.

6. Satz von Nukleinsäuren nach Anspruch 4, dadurch **gekennzeichnet**, daß der Satz aus der Gruppe von Nukleinsäurezusammensetzungs-Sätzen, die durch die folgenden Sonden definiert sind:
Sonde 2191 und Sonde 2228;
Sonde 2228 und Sonde 2260;
Sonde 2191 und Sonde 2260; und ihre komplementären Sonden ausgewählt ist, wobei der Satz in der Lage ist, Mycoplasma hominis nachzuweisen.

7. Satz von Nukleinsäuren nach Anspruch 4, dadurch **gekennzeichnet,** daß der Satz aus der Gruppe von Nukleinsäurezusammensetzungs-Sätzen, die durch die Sonde 2227 und die Sonde 2219 und deren komplementäre Sonden definiert werden, ausgewählt ist, dadurch **gekennzeichnet,** daß der Satz in der Lage ist, Mycoplasma genitalium nachzuweisen.

8. Satz von Nukleinsäuren nach Anspruch 4, dadurch **gekennzeichnet,** daß der Satz aus der Gruppe von Nukleinsäurezusammensetzungs-Sätzen, die durch die folgenden Sonden definiert sind:
Sonde 2335 und Sonde 2337;
Sonde 2335 und Sonde 2334;
Sonde 2334 und Sonde 2336 und deren komplementäre Sonden ausgewählt ist, dadurch **gekennzeichnet,** daß der Satz in der Lage ist, Mycoplasma genitalium nachzuweisen.

9. Verfahren zum Nachweis von Mycoplasma hominis, Ureaplasma urealyticum oder Mycoplasma genitalium in einer Probe, umfassend:
a) Inkontaktbringen der Probe mit wenigstens einer Nukleinsäurezusammensetzung mit 10 bis 250 Nukleotiden, die, unter Bedingungen, die der Nukleinsäurezusammensetzung ermöglichen, vorzugsweise an rRNA oder rDNA des Mycoplasmen-Organismus zu hybridisieren, wobei die Nukleinsäure mit wenigstens einer Region der rRNA oder rDNA, ausgewählt aus den Bereichen bestehend aus:
Positionen 50 bis 100, 425 bis 485 oder 1100 bis 1150 der Mycoplasma hominis 16S rRNA;
Positionen 50 bis 100, 150 bis 250, 425 bis 485, 800 bis 850, 1090 bis 1160 oder 1220 bis 1260 der Ureaplasma urealyticum 16S rRNA;
Positionen 1110 bis 1160 der Mycoplasma genitalium 16S rRNA; und
Positionen 260 bis 330, 1590 bis 1630 oder 1850 bis 1900 der Mycoplasma genitalium 23S rRNA, hybridisiert,
b) Anwenden von Hybridisierungsbedingungen auf die Probe, so daß ein Hybridisierungsprodukt in Gegenwart einer Zielsequenz gebildet wird und
c) Nachweis des Hybridisierungsproduktes als Hinweis auf das Vorhandensein der Mycoplasmen.

10. Verfahren nach Anspruch 9, dadurch **gekennzeichnet**, daß die Nukleinsäurezusammensetzung der Stufe des Inkontaktbringens komplementär oder homolog zu wenigstens 90% von irgendwelchen zehn aufeinanderfolgenden Nukleotiden, ausgewählt aus der Gruppe von Sequenzen, die durch die Sonden, bestehend aus 2219, 2262, 2256, 2246, 2218, 2271, 2220, 2259, 2227, 2335, 2337, 2334, 2336, 2191, 2228 und 2260, definiert werden, ist.

11. Verfahren nach Anspruch 9, dadurch **gekennzeichnet**, daß die Stufe des Inkontaktbringens eine erste Nukleinsäurezusammensetzung und eine zweite Nukleinsäurezusammensetzung verwendet, dadurch **gekennzeichnet**, daß die ersten und zweiten Nukleinsäuren verschiedene Sequenzen haben, die komplementär oder homolog zu wenigstens 90% von irgendwelchen zehn aufeinanderfolgenden Nukleotiden, ausgewählt aus der Gruppe von Sequenzen, definiert durch die Sonden, bestehend aus 2219, 2262, 2256, 2246, 2218, 2271, 2220, 2259, 2227, 2335, 2337, 2334, 2336, 2191, 2228 und 2260, sind.

12. Verfahren nach Anspruch 11, dadurch **gekennzeichnet**, daß die erste Nukleinsäurezusammensetzung und die zweite Nukleinsäurezusammensetzung Sätze sind, die aus der Gruppe von Sätzen ausgewählt sind, die durch Sonden, bestehend aus:
Sonde 2262 und Sonde 2256;
Sonde 2246 und Sonde 2218;
Sonde 2259 und Sonde 2271;
Sonde 2271 und Sonde 2220;
Sonde 2191 und Sonde 2228;
Sonde 2228 und Sonde 2260;
Sonde 2191 und Sonde 2260;
Sonde 2227 und Sonde 2219;
Sonde 2335 und Sonde 2337;
Sonde 2335 und Sonde 2334; und
Sonde 2334 und Sonde 2336 definiert sind.

13. Kit zum Nachweis des Vorhandenseins eines Organismus oder mehrerer Organismen, ausgewählt aus Mycoplasma hominis, Ureaplasma urealyticum und Mycoplasma genitalium, wobei der Kit eine Nukleinsäure mit 10 bis 250 Nukleotiden, die in der Lage sind, vorzugsweise an rRNA oder rDNA des Organismus zu hybridisieren, umfaßt, wobei die Nukleinsäure mit wenigstens einem Bereich der rRNA oder rDNA, ausgewählt aus den Regionen bestehend aus:
Positionen 50 bis 100, 425 bis 485 oder 1100 bis 1150 der Mycoplasma hominis 16S rRNA;
Positionen 50 bis 100, 150 bis 250, 425 bis 485, 800 bis 850, 1090 bis 1160 oder 1220 bis 1260 der Ureaplasma urealyticum 16S rRNA;
Positionen 1110 bis 1160 der Mycoplasma genitalium 16S rRNA; und
Positionen 260 bis 330, 1590 bis 1630 oder 1850 bis 1900 der Mycoplasma genitalium 23S rRNA hybridisiert.

## Revendications

1. Acide nucléique contenant approximativement de 10 à 250 nucléotides, apte à s'hybrider de manière préférentielle à l'ARNr ou à l'ADNr de bactéries mycoplasmiques pathogènes choisies parmi Mycoplasma hominis, Ureaplasma urealyticum et Mycoplasma genitalium qui sont associées au tractus urinaire humain et aux zones génitales, dans lequel ledit acide nucléique s'hybride à au moins une région de l'ARNr ou de l'ADNr choisie parmi les régions constituées par:
les positions 50 à 100, 425 à 485 ou 1100 à 1150 de l'ARNr 16S de Mycoplasma hominis;
les positions 50 à 100, 150 à 250, 425 à 485, 800 à 850, 1090 à 1160 ou 1220 à 1260 de l'ARNr 16S d'Ureaplasma urealyticum;
les positions 1110 à 1160 de l'ARNr 16S de Mycoplasma genitalium; et
les positions 260 à 330, 1590 à 1630 ou 1850 à 1900 de l'ARNr 23S de Mycoplasma genitalium.

2. Acide nucléique selon la revendication 1, dans lequel ledit acide nucléique est complémentaire à concurrence d'au moins 90% d'une séquence comprenant dix nucléotides consécutifs quelconques dont la séquence d'acides nucléiques est choisie parmi le groupe constitué par les séquences 2262, 2256, 2246, 2218, 2271, 2220, 2259, 2227, 2335, 2337, 2334, 2336, 2191, 2228 et 2260.

3. Acide nucléique selon la revendication 1, dans lequel ledit acide nucléique est homologue à concurrence d'au moins 90% d'une séquence comprenant dix nucléotides consécutifs quelconques dont la séquence d'acides nucléiques est choisie parmi le groupe constitué par les séquences définies par des sondes constituées par les sondes 2262, 2256, 2246, 2218, 2271, 2220, 2259, 2227, 2335, 2337, 2334, 2336, 2191, 2228 et 2260.

4. Ensemble d'au moins deux compositions d'acides nucléiques, chaque composition d'acide nucléique contenant approximativement de 10 à 250 nucléotides de composition séquentielle différente, apte à s'hybrider de manière préférentielle à l'ARNr ou à l'ADNr de bactéries mycoplasmiques pathogènes choisies parmi Mycoplasma hominis, Ureaplasma urealyticum et Mycoplasma genitalium qui sont associées au tractus urinaire humain et aux zones génitales, dans lequel chaque acide nucléique est complémentaire ou homologue à concurrence d'au moins 90% d'une séquence comprenant dix nucléotides consécutifs quelconques choisie parmi le groupe de séquences définies par les sondes 2262, 2256, 2246, 2218, 2271, 2220, 2259, 2227, 2335, 2337, 2334, 2336, 2191, 2228 et 2260, ledit acide nucléique restant étant choisi parmi les séquences restantes.

5. Ensemble d'acides nucléiques selon la revendication 4, dans lequel ledit ensemble est choisi parmi le groupe d'ensembles de compositions d'acides nucléiques définis par les sondes:
sonde 2262 et sonde 2256;
sonde 2246 et sonde 2218;
sonde 2259 et sonde 2271;
sonde 2271 et sonde 2220; et leurs compléments, dans lequel ledit ensemble est capable de détecter Ureaplasma urealyticum.

6. Ensemble d'acides nucléiques selon la revendication 4, dans lequel ledit ensemble est choisi parmi le groupe d'ensembles de compositions d'acides nucléiques définis par les sondes:
sonde 2191 et sonde 2228;
sonde 2228 et sonde 2260;
sonde 2191 et sonde 2260; et leurs compléments, dans lequel ledit ensemble est capable de détecter Mycoplasma hominis.

7. Ensemble d'acides nucléiques selon la revendication 4, dans lequel ledit ensemble est choisi parmi le groupe d'ensembles de compositions d'acides nucléiques définis par la sonde 2227 et par la sonde 2219, ainsi que par leurs compléments, dans lequel ledit ensemble est capable de détecter Mycoplasma genitalium.

8. Ensemble d'acides nucléiques selon la revendication 4, dans lequel ledit ensemble est choisi parmi le groupe d'ensembles de compositions d'acides nucléiques définis par les sondes:
sonde 2335 et sonde 2337;
sonde 2335 et sonde 2334;
sonde 2334 et sonde 2336; et leurs compléments, dans lequel ledit ensemble est capable de détecter Mycoplasma genitalium.

9. Procédé pour détecter Mycoplasma hominis, Ureaplasma urealyticum ou Mycoplasma genitalium dans un échantillon, comprenant le fait de:
a) mettre en contact ledit échantillon avec au moins une composition d'acide nucléique possédant de 10 à 250 nucléotides dans des conditions qui permettent à ladite composition d'acide nucléique de s'hybrider de manière préférentielle à l'ARNr ou à l'ADNr dudit organisme mycoplasmique, dans lequel ledit acide nucléique s'hybride à au moins une région de l'ARNr ou de l'ADNr choisie parmi des régions constituées par:
les positions 50 à 100, 425 à 485 ou 1100 à 1150 de l'ARNr 16S de Mycoplasma hominis;
les positions 50 à 100, 150 à 250, 425 à 485, 800 à 850, 1090 à 1160 ou 1220 à 1260 de l'ARNr 16S d'Ureaplasma urealyticum;
les positions 1110 à 1160 de l'ARNr 16S de Mycoplasma genitalium; et
les positions 260 à 330, 1590 à 1630 ou 1850 à 1900 de l'ARNr 23S de Mycoplasma genitalium;
b) imposer des conditions d'hybridation audit échantillon pour former un produit d'hybridation en présence d'une cible; et
c) détecter ledit produit d'hybridation comme étant une indication de la présence desdits mycoplasmes.

10. Procédé selon la revendication 9, dans lequel ladite composition d'acide nucléique de ladite étape de mise en contact est complémentaire ou homologue à concurrence d'au moins 90% de dix nucléotides consécutifs quelconques choisis parmi le groupe de séquences définies par des sondes constituées par les sondes 2219, 2262, 2256, 2246, 2218, 2271, 2220, 2259, 2227, 2335, 2337, 2334, 2336, 2191, 2228 et 2260.

11. Procédé selon la revendication 9, dans lequel, dans ladite étape de mise en contact, on utilise une première composition d'acides nucléiques et une seconde composition d'acides nucléiques, lesdits premier et second acides nucléiques possédant des séquences différentes, qui sont complémentaires ou homologues à concurrence d'au moins 90% de dix nucléotides consécutifs quelconques choisis parmi le groupe de séquences définies par des sondes constituées par les sondes 2219, 2262, 2256, 2246, 2218, 2271, 2220, 2259, 2227, 2335, 2337, 2334, 2336, 2191, 2228 et 2260.

12. Procédé selon la revendication 1, dans lequel ladite première composition d'acides nucléiques et ladite seconde composition d'acides nucléiques sont des ensembles choisis parmi le groupe d'ensembles définis par des sondes constituées par les sondes:
sonde 2262 et sonde 2256;
sonde 2246 et sonde 2218;
sonde 2259 et sonde 2271;
sonde 2271 et sonde 2220;
sonde 2191 et sonde 2228;
sonde 2228 et sonde 2260;
sonde 2191 et sonde 2260;
sonde 2227 et sonde 2219;
sonde 2335 et sonde 2337;
sonde 2335 et sonde 2334; et
sonde 2334 et sonde 2336.

13. Nécessaire pour détecter la présence d'un ou de plusieurs organismes choisis parmi Mycoplasma hominis, Ureaplasma urealyticum et Mycoplasma genitalium, qui comprend un acide nucléique contenant de 10 à 250 nucléotides aptes à s'hybrider de manière préférentielle à l'ARNr ou à l'ADNr desdits organismes, dans lequel ledit acide nucléique s'hybride à au moins une région de l'ARNr ou de l'ADNr choisie parmi les régions constituées par:
les positions 50 à 100, 425 à 485 ou 1100 à 1150 de l'ARNr 16S de Mycoplasma hominis;
les positions 50 à 100, 150 à 250, 425 à 485, 800 à 850, 1090 à 1160 ou 1220 à 1260 de l'ARNr 16S d'Ureaplasma urealyticum;
les positions 1110 à 1160 de l'ARNr 16S de Mycoplasma genitalium; et
les positions 260 à 330, 1590 à 1630 ou 1850 à 1900 de l'ARNr 23S de Mycoplasma genitalium.
